Europäisches Patentamt

European Patent Office

Office européen des brevets

(11) Numéro de publication: **0 362 450**
**A1**

## (12) DEMANDE DE BREVET EUROPEEN

(21) Numéro de dépôt: **88402543.8**

(51) Int. Cl.5: **A61K 7/48 , A61K 31/19**

(22) Date de dépôt: **07.10.88**

(43) Date de publication de la demande:
**11.04.90 Bulletin 90/15**

(84) Etats contractants désignés:
**AT BE CH DE ES FR GB GR IT LI LU NL SE**

(71) Demandeur: **Reckitt & Colman S.A.**
**15, rue Ampère**
**F-91301 Massy Cédex(FR)**

Demandeur: **Jean, Daniel**
**Rue de la Chaussade**
**F-63270 Vic-le-Comte(FR)**

(72) Inventeur: **Cariel, Léon**
**14, rue Raynouard**
**F-75016 Paris(FR)**
Inventeur: **Jean, Daniel**
**Rue de la Chaussade**
**F-63270 Vic-le-Comte(FR)**

(74) Mandataire: **Rinuy, Santarelli**
**14, avenue de la Grande Armée**
**F-75017 Paris(FR)**

(54) **Composition pharmaceutique et/ou cosmétique à activité régulatrice de la pigmentation cutanée.**

(57) La présente invention concerne une composition pharmaceutique et/ou cosmétique, à activité régulatrice de la pigmentation cutanée, caractérisée en ce qu'elle comprend à titre de substance active, un composé chimique ayant une activité inhibitrice de la peroxydase et de la tyrosinase.

Cette composition est disponible sous formes de lotion, de lait, de stick, de masque, de gel ou de crème.

EP 0 362 450 A1

## COMPOSITION PHARMACEUTIQUE ET/OU COSMETIOUE A ACTIVITE REGULATRICE DE LA PIGMENTATION CUTANEE.

La présente invention concerne une composition pharmaceutique et/ou cosmétique à activité régulatrice de la pigmentation cutanée.

Le mécanisme de formation du pigment responsable de la coloration de la peau, la mélanine, est un mécanisme complexe d'oxydation qui répond, très schématiquement, au schéma suivant.

Tyrosine→Dopa→Dopachrome→mélanine.

Cette suite de réactions fort complexes est soumise à l'action de la tyrosinase qui catalyse l'oxydation des différents substrats. Il est tout à fait possible de réaliser une biosynthèse de mélanine naturelle "in vitro" en partant de la tyrosine et de la tyrosinase.

Ces oxydations toutefois nécessitent un apport important d'oxygène dont le transfert peut être assuré efficacement par une peroxydase qui va dégrader les peroxydes formés dans le derme lors des processus oxydatifs respiratoires.

Les peaux naturellement colorées, les peaux blanches ainsi que celles qui ont subi l'action de rayonnements ultra-violets présentent parfois des tâches brunes irrégulières. Ces taches peuvent être dues à la sénescence, à un effet irrégulier des radiations solaires, à la grossesse, à l'utilisation de contraceptifs oraux, à l'utilisation de produits solaires mals tolérés, au contact avec des plantes ou des extraits de plantes contenant des psoralènes ou d'autres substances photosensibilisantes.

Ces irrégularités dans la pigmentation cutanée semblent dues à une distribution anarchique de l'oxygène disponible pour les étapes d'oxydation de la tyrosine en mélanine.

La présente invention a pour but de pallier l'inconvénient cité plus haut, en proposant une composition pharmaceutique et/ou cosmétique à activité régulatrice de la pigmentation cutanée caractérisée en ce qu'elle comprend, à titre de substance active, au moins un composé chimique ayant une activité inhibitrice de la peroxydase et de la tyrosinase.

Plusieurs composés chimiques possèdent cette activité inhibitrice de la peroxydase et de la tyrosinase, en particulier l'acide gallique et le pyrogallol. Dans le cadre de l'invention, on préfère utiliser une composition à usage topique externe présentant toutes les garanties d'innocuité. Une composition qui comporte à titre de substance active l'acide gallique ou l'un de ses esters, notamment avec des alcools en $C_3$ à $C_9$, présente ces caractéristiques.

Selon une caractéristique particulière de l'invention, ledit composé chimique est l'acide gallique.

Selon une autre caractéristique, ladite composition pharmaceutique et/ou cosmétique contient environ 0,2 % à environ 2,5 % en poids d'acide gallique ou de ses esters.

La composition selon l'invention comporte en outre des substances utiles pour sa mise en forme galénique ainsi qu'éventuellement un agent favorisant la pénétration du composé actif dans la peau.

La composition selon l'invention convient pour les peaux naturellement colorées, les peaux blanches ainsi que pour celles qui ont subi l'action des rayonnements ultraviolets.

Les esters de l'acide gallique, notamment l'ester de méthyle, butyle, lauryle par exemple, présentent l'avantage d'une stabilité améliorée par rapport à l'acide gallique lui-même, notamment l'ester de butyle qui tout en présentant une bonne stabilité garde une certaine solubilité dans l'eau et présente des propriétés pharmacologiques voisines de l'acide libre.

Il est bien entendu possible d'utiliser d'autres dérivés de la fonction acide ou des esters différents de ceux décrits précédemment pour améliorer les propriétés des compositions.

D'autres caractéristiques et avantages de la présente invention apparaîtront à la lecture de la description qui va suivre.

On a cherché à évaluer dans un premier temps l'effet inhibiteur sur la tyrosinase de différentes substances. Le mode opératoire est décrit dans l'exemple qui suit :

### EXEMPLE 1 :

La tyrosinase utilisée a été extraite de carpophores de Psalliota bispora frais par un tampon phosphate à pH 6,8.

Le dosage de l'activité tyrosinase est effectué en mesurant à 475 nm à 25° C l'évolution de la D.O. de la solution suivante :

| Tampon pH 6,8 .... | 0,5 ml |
| DOPA à 0,1 % dans du tampon pH 6,8.... | 1 ml |
| Solution enzymatique à doser .... | 1 ml |

L'évaluation de l'activité d'un inhibiteur est effectué en dissolvant la substance à tester dans le tampon à une concentration de 0,02 % (0,1 mg dans 0,5 ml de tampon).

Les mesures sont effectuées par rapport à une solution témoin ne compartant pas de substance à tester.

Plusieurs composés ont été testés, en particulier l'hydroquinone et la vitamine C, deux substances commercialisées, qui sont connues pour avoir un effet sur la pigmentation. Le tableau I suivant illustre les résultats obtenus.

## TABLEAU I

| SUBSTANCE TESTEE | POUVOIR INHIBITEUR[*] (%) |
|---|---|
| Hydroquinone | + 4,5 |
| Vitamine C | - 10 |
| Acide Kojique | + 88 |
| Acide cafféique | - 32 |
| Acide gallique | + 45 |
| Pyrogallol | + 45,1 |
| 3-hydroxy-2-méthyl-4 pyrone | O |
| Acide azélaique | 3 |

$$* \text{ Pouvoir inhibiteur} = \frac{\text{DO solution testée}}{\text{DO solution témoin}} \times 100$$

On constate que les deux produits commercialisés ne figurent pas parmi les substances les plus actives, la vitamine C possédant même un pouvoir activateur de la tyrosinase.

Dans un second temps, on a évalué l'effet inhibiteur sur la peroxydase de certaines substances.

## EXEMPLE II

La peroxydase utilisée a été extraite de racines fraiches de Cochlearia Armoracia par du tampon pH 6,8.

Le dosage de l'activité peroxydasique se fait en mesurant l'évolution de la D.O. à 485 nm de la solution suivantes :

3

| Tampon pH 6,8.... | 1,5 ml |
|---|---|
| Peroxyde d'hydrogène 10 vol. .... | 0,1 ml |
| Solution à 1 % dans du tampon pH 6,8 de p. phénylènediamine 0,1 ml .... | 0,1 ml |
| Solution enzymatique à doser .... | 0,4 ml. |

L'évaluation du pouvoir inhibiteur d'une substance est mesuré en dissolvant cette substance dans le tampon à la concentration de 0,005 % (75 µg dans 1,5 ml de tampon).

Les composés utilisés dans l'exemple I ont de nouveau été testés.

TABLEAU II

| SUBSTANCE TESTEE | : | POUVOIR INHIBITEUR |
|---|---|---|
| Hydroquinone | : | + 36,5 |
| Vitamine C | : | + 6,5 |
| Acide Kojique | : | - 20,5 |
| Acide cafféique | : | + 43,5 |
| Acide gallique | : | + 51 |
| Pyrogallol | : | + 83 |
| 3 hydroxy-2-methyl-4 pyrone | : | + 2,5 |
| Acide azelaique | : | - 4 |

Il apparaît, à la lecture des deux tableaux précédents que le pyragallol et l'acide gallique ont un effet inhibiteur fort, à la fois sur la tyrosinase et la peroxydase.

Toutefois, le pyrogallol est un produit mal toléré par la peau. Par conséquent, il a été écarté de cette étude.

EXEMPLE III

Etude clinique de l'acide gallique en tant que régulateur de la pigmentation cutanée.

On sélectionne cinquante femmes dont l'âge est compris entre 18 et 45 ans et présentant un chloasma persistant (Le chloasma est un ensemble de taches du visage. Il survient au visage de la femme enceinte et constitue le masque de grossesse) depuis plus de deux ans après une grossesse ou pendant l'usage permanent de contraceptifs oraux.

On sélectionne aussi 15 personnes dont l'âge est compris entre 24 et 55 ans, 7 hommes et 8 femmes, présentant des taches au visage consécutives à l'utilisation de produits solaires.

Enfin, on sélectionne 20 personnes dont l'âge est compris entre 45 et 78 ans, dix hommes et dix femmes présentant des taches hypermélaniques aux mains.

Dans tous les cas, on traite une partie de la zone atteinte par une solution placebo et une partie équivalente conjointe et nettement délimitée sur des photographies par une solution à 0,5 % d'acide gallique dans de l'alcool à 40 %, à usage topique externe.

Le traitement intervient deux fois par jour, matin et soir, pour l'essai et pour le placebo et ce pendant un mois.

L'évaluation de l'efficacité est déterminée par une méthode réflectométrique consistant à mesurer la lumière réfléchie par la peau hyperpigmentée en prenant la peau saine du même sujet comme référence.

Cette mesure fait intervenir le tracé du spectre de la lumière réfléchie de 400 à 800 nm ce qui permet de différencier un simple érythème d'une véritable hyperpigmentation.

On obtient donc le tracé de trois courbes : une pour la peau saine, une pour la peau hyperpigmentée, et une pour l'intensité relative des deux courbes.

On réalise une mesure au début de l'expérimentation, une après un mois de traitement et une dernière

4

après trois mois de traitement.

## Résultats

Dans presque tous les cas, une amélioration très sensible est obtenue le premier mois.

## Chloasma :

On note une disparition totale de l'irrégularité de la pigmentation dans 44 cas sur 50, soit dans 88 % des cas.
On note une légère amélioration dans 2 cas et pas de résultat du tout dans 4 cas.

## Taches dues à l'utilisation de produits solaires

On note une disparition totale des taches dans 11 cas sur 15 soit dans 73 % des cas.
On note une amélioration sensible dans un cas et pas d'amélioration du tout dans 3 cas.

## Taches de sénescence

On note une amélioration sensible des taches dans 12 cas sur 20 soit dans 60 % des cas.
On note une légère amélioration dans 3 cas et pas d'amélioration du tout dans 5 cas.
On ne note aucune disparition totale des taches.
Dans aucun des cas étudiés, on n'observe d'intolérance ou de gène au traitement.
L'acide gallique convient pour la préparation de compositions galéniques différentes. Ces compositions sont mises en oeuvre selon des méthodes classiques. L'exemple suivant en illustre quelques unes.

## EXEMPLE 4 :

| Formulation de lotion (% en poids) | |
|---|---|
| Glycérol .... | 8 à 12 |
| Acide gallique .... | 0,2 à 0,8 |
| NaOH .... | 0,05 à 0,15 |
| Eau, parfum, conservateurs .... | complément à 100 %. |

| Formulation de gel (% en poids) | |
|---|---|
| Acide polyacrylique réticulé .... | 0,5 à 1 |
| Acide gallique .... | 0,2 à 0,8 |
| NaOH .... | 0,1 à 0,3 |
| Eau,parfum, conservateurs .... | complément à 100 %. |

| Formulation de crème (% en poids) | |
|---|---|
| Stéarate de glycérol .... | 12 à 18 |
| Cétostéarate de polyoxyéthylène glycol.... | 2 à 4 |
| di-n-butyladipate .... | 8 à 12 |
| huile de paraffine .... | 2 à 4 |
| Glycérol .... | 4 à 5 |
| Acide gallique .... | 0,5 à 1,5 |
| NaOH .... | 0,1 à 0,3 |
| Eau, parfum, conservateurs .... | complément à 100 %. |

| Formulation de lait (% en poids) | |
|---|---|
| Mélange de mono et diglycérides d'acide palmitostéarique .... | 5 à 7 |
| Alcool cétostéarylique éthoxylé à 12 molécules d'oxyde d'éthylène.... | 1 à 2 |
| Alcool cétostéarylique éthoxylé à 20 molécules d'oxyde d'éthylène .... | 1 à 2 |
| 2-octy-dodécanol .... | 8 à 12 |
| Triglycéride d'acide caprique et caprylique .... | 4 à 6 |
| Huile de paraffine visqueuse .... | 4 à 6 |
| Acide gallique .... | 0,2 à 0,8 |
| Eau, parfum, conservateurs .... | complément à 100 %. |

| Formulation de lotion à base d'acide gallique à forte concentration (% en poids) | |
|---|---|
| Monopropylène glycol .... | 8 à 12 |
| Acide gallique .... | 1,5 à 2,5 |
| Polyolester d'acide gras .... | 4 à 6 |
| Eau, parfum, conservateurs .... | complément à 100 % |

| Formulation de masque (% en poids) | |
|---|---|
| Alginates de sodium .... | 1,5 à 2,5 |
| Alcool éthylique à 96 % .... | 8 à 12 |
| Fécule de riz .... | 27 à 33 |
| Oxyde de zinc .... | 2 à 4 |
| Acide gallique .... | 0,8 à 1,3 |
| Eau, parfum, conservateurs .... | complément à 100 % |

| Formulation de stick (% en poids) | |
|---|---|
| Alcool cétostéarylique .... | 2 à 4 |
| 2-octyl-dodécanol .... | 40 à 46 |
| Monoéthanolamide d'acides gras de coprah. | 2 à 4 |
| Acide palmitostéarique .... | 9 à 9,6 |
| Huile de paraffine visqueuse .... | 2,2 à 3,5 |
| Alcool éthylique à 96 % .... | 37,7 à 38,5 |
| Acide gallique .... | 0,2 à 0,8 |

**Revendications**

1. Application d'un composé inhibant la peroxydase et la tyrosinase pour obtenir une composition pharmaceutique et/ou cosmétique destinée à régulariser la pigmentation cutanée, notamment dans les cas de chloasma, de taches dues à l'utilisation de produits solaires et les taches de sénescence.

2. Application selon la revendication 1, caractérisée en ce que le composé inhibant la peroxydase et la tyrosinase est l'acide gallique ou un ester de l'acide gallique.

3. Application selon la revendication 2, caractérisée en ce que le composé est un ester de l'acide gallique en $C_3$ à $C_9$.

4. Application selon la revendication 3, caractérisée en ce que le composé est le gallate de butyle.

5. Application selon l'une des revendications 1 à 4, caractérisée en ce que la composition comporte 0,2 à 2,5 % en poids du composé inhibant la peroxydase et la tyrosinase.

6. Application selon l'une des revendications 1 à 5, caractérisée en ce que la composition est destinée à un usage topique externe.

7. Application selon l'une des revendications 1 à 6, caractérisée en ce que la composition est disponible sous forme de lotion, de lait, de stick, de masque, de gel ou de crème.

8. Application selon l'une des revendications 1 à 7, caractérisée en ce qu'elle répond à la formulation suivante (pourcentages en poids) :

| Glycérol .... | 8 à 12 % |
|---|---|
| Acide gallique ou ester .... | 0,2 à 0,8 % |
| NaOH .... | 0,005 à 0,15 % |
| Eau, parfum, conservateurs .... | complément à 100 % |

9. Application selon l'une des revendications 1 à 7, caractérisée en ce que la composition répond à la formulation suivante (pourcentages en poids) :

| Stéarate de glycérol .... | 12 à 18 % |
|---|---|
| Cétostéarate de polyoxyéthylène glycol .... | 2 à 4 % |
| di-n-butyladipate .... | 8 à 12 % |
| huile de paraffine .... | 2 à 4 % |
| Glycérol .... | 4 à 5 % |
| Acide gallique ou ester .... | 0,5 à 1,5 % |
| NaOH .... | 0,1 à 0,3 % |
| Eau, parfum, conservateurs .... | complément à 100 %. |

10. Application selon l'une des revendications 1 à 7, caractérisée en ce que la composition répond à la formulation suivante (pourcentages en poids) :

| | |
|---|---|
| Mélange de mono et diglycérides d'acide palmitostéarique .... | 5 à 7 % |
| Alcool cétostéarylique éthoxylé à 20 molécules d'oxyde d'éthylène .... | 1 à 2 % |
| Alcool cétostéarylique éthoxylé à 20 molécules d'oxyde d'éthylène .... | 1 à 2 % |
| 2-octy-dodécanol .... | 8 à 12 % |
| Triglycéride d'acide caprique et caprylique .... | 4 à 6 % |
| Huile de paraffine visqueuse .... | 4 à 6 % |
| Acide gallique ou ester .... | 0,2 à 0,8 % |
| Eau, parfum, conservateurs .... | complément à 100 %. |

11. Application selon l'une des revendications 1 à 7, caractérisée en ce que la composition répond à la formule suivante (pourcentage en poids).

| | |
|---|---|
| Monopropylène glycol .... | 8 à 12 % |
| Acide gallique ou ester .... | 1,5 à 2,5 % |
| Polyolester d'acide gras .... | 4 à 6 % |
| Eau, parfum, conservateurs .... | complément à 100 % |

12. Application selon l'une des revendications 1 à 7, caractérisée en ce que la composition répond à la formulation suivante (pourcentage en poids).

| | |
|---|---|
| Alginates de sodium .... | 1,5 à 2,5 % |
| Alcool éthylique à 96 % .... | 8 à 12 % |
| Fécule de riz .... | 27 à 33 % |
| Oxyde de zinc .... | 2 à 4 % |
| Acide gallique ou ester .... | 0,8 à 1,3 % |
| Eau, parfum, conservateurs .... | complément à 100 %. |

13. Application selon l'une des revendications 1 à 7, caractérisée en ce que la composition répond à la formulation suivante (pourcentages en poids) :

| | |
|---|---|
| Alcool cétostéarylique .... | 2 à 4 % |
| 2-octyl-dodécanol .... | 40 à 46 % |
| Monoéthanolamide d'acides gras de coprah .... | 2 à 4 % |
| Acide palmitostéarique .... | 9 à 9,6 % |
| Huile de paraffine visqueuse .... | 2,2 à 3,5 % |
| Alcool éthylique à 96 % .... | 37,7 à 38,5 % |
| Acide gallique ou ester .... | 0,2 à 0,8. |

Office européen
des brevets

**RAPPORT DE RECHERCHE EUROPEENNE**

Numero de la demande

EP 88 40 2543

## DOCUMENTS CONSIDERES COMME PERTINENTS

| Catégorie | Citation du document avec indication, en cas de besoin, des parties pertinentes | Revendication concernée | CLASSEMENT DE LA DEMANDE (Int. Cl.5) |
|---|---|---|---|
| X | PATENT ABSTRACTS OF JAPAN, vol. 12, no. 82 (C-481)[2929], 15th March 1988; & JP-A-62 215 512 (POLA CHEM. IND. INC.) 22-09-1987 * Résumé * --- | 1-3,5,6 ,7 | A 61 K 7/48 A 61 K 31/19 |
| A | CHEMICAL ABSTRACTS, vol. 85, no. 24, 13 décembre 1976, page 308, résumé no. 182272a, Columbus, Ohio, US; & JP-A-76 101 138 (ICHIMARU CO. LTD) 07-09-1976 * Résumé * --- | 1 | |
| A | HAGERS HANDBUCH DER PHARMAZEUTISCHEN PRAXIS, vol. 6, partie C, pages 274-275, Springer-Verlag, Berlin, DE * Page 274, "Trihydroxy-benzoesäure" - page 275, paragraphe 1 * --- | 1,2 | |
| A | THE MERCK INDEX, 10ième édition, 1983, pages 621, 1154, Merck & Co., Inc., Rahway, N.J., US * No. 4216, no. 7901 * --- | 1-3 | |
| A | MARTINDALE - THE EXTRA PHARMACOPOEIA, 28ième édition, 1982, pages 501,1282, The Pharmaceutical Press, Londres, GB * Page 501: "Pyrogallol"; page 1282: "The Alkyl Gallates" * --- | 1-4 | DOMAINES TECHNIQUES RECHERCHES (Int. Cl.5) A 61 K |
| E | FR-A-2 613 622 (L. CARIEL et al.) * En entier * ----- | 1-13 | |

Le présent rapport a été établi pour toutes les revendications

| Lieu de la recherche | Date d'achèvement de la recherche | Examinateur |
|---|---|---|
| LA HAYE | 27-06-1989 | WILLEKENS G.E.J. |